# EUROPEAN PATENT SPECIFICATION

(11) **EP 1 585 491 B1**
(45) Date of publication and mention of the grant of the patent: **22.10.2014**
(21) Application number: 04701005.3
(22) Date of filing: 09.01.2004
(51) Int. Cl.: A61K 8/26, A61Q 11/00, A61K 8/19, A61K 8/22

(54) **ORAL COMPOSITION**
ORALE ZUSAMMENSETZUNG
COMPOSITION BUCCO-DENTAIRE

(30) Priority: 22.01.2003 EP 03075233
(43) Date of publication of application: 19.10.2005
(73) Proprietor: Unilever N.V., 3013 AL Rotterdam (NL); Unilever PLC, London, Greater London EC4Y 0DY (GB)
(72) Inventor: LEFORT, Stephane, c/o Lever Faberge Italia S.r.l., I-26841 Casalpusterlengo (IT); RAVIDA, Nunziatino, c/o Lever Faberge Italia S.r.l, I-26841 Casalpusterlengo (IT); PICKLES, Matthew Jonathan Unilever R&D Port Sun., Bebington, Wirral Mersey CH63 3JW (GB)
(74) Representative: Tansley, Sally Elizabeth
(86) International application number: PCT/EP2004/000145
(87) International publication number: WO 2004/064795

(56) References cited:
- EP-A- 1 051 962
- EP-A2- 0 219 483
- WO-A1-03/030850
- US-A- 5 597 553
- US-A- 5 976 506
- US-B1- 6 355 227
- US-B1- 6 432 387
- LUTZ F ET AL: "SELF-ADJUSTING ABRASIVENESS: A NEW TECHNOLOGY FOR PROPHYLAXIS PASTES", QUINTESSENCE INTERNATIONAL, QUINTESSENZ VERLAG, BERLIN, DE, vol. 24, no. 1, 1 January 1993 (1993-01-01), pages 53-63, XP008024874, ISSN: 0033-6572

## Description

The invention provides an oral composition with improved abrasive effect.

WO 02/45676 (Hawe Neos) discloses a cleaning agent with an abrasive component for cleaning delicate surfaces especially teeth. This is preferably achieved through the use of components such as glass flakes.

EP 0528756 (Hawe Neos) discloses a dental care and cleaning composition which is substantially free from water. This composition comprises a finely divided rock which may be in the form of perlite and 1,2-propanediol and at least one wetting agent for the rock particles. This requires finely divided rock that has been put through an expensive sieving technique to ensure that it is a uniform composition, and that they are small enough to effectively clean while not causing excessive abrasion.

US 5124143 (Muhlemann) discloses a dentrifice comprising silicic acid and perlite. This combination provides for unexpectedly high abrasiveness in order to allow for a paste with preventative dental hygiene properties for use by a dental hygienist.

US 5 976 506 discloses oral compositions comprising calcium carbonate and perlite containing agglomerates.

US 6355227 (Unilever) discloses an oral composition comprising 0.01 to 0.9% perlite w/w.

WO 99/31184 (Crosfield) discloses a liquid product incorporating a material in the form of granules of high density inorganic particles co-agglomerated with particles having a density lower than water in order to tailor the density of the granules and in order to aid their buoyancy.

Despite the presence in the prior art of such oral compositions as outlined above, there is still a need for an oral composition which is capable of providing improved polishing while not increasing the abrasivity, or relative dental abrasion (RDA) of such an oral composition.

It has been found that the provision of perlite and chalk together in an oral composition aids in the polishing of teeth. This combination increases the oral composition's ability to remove adherent soiling matter, to free accessible plaque, to dislodge accessible debris and to remove superficial stain from the teeth which in turn leads to improved mouthfeel and teeth whitening benefits.

According to the present invention there is provided an oral composition according to claim 1 comprising chalk characterised in that the oral composition comprises perlite.

Perlite comprises flat or relatively planar particles which, when ground consist of jagged-edged, eggshell-shaped fragments which are broken down during their use into smaller fragments.

The physical structure of the perlite means that when the perlite particles are subjected to pressure, for example by a toothbrush during brushing, their planar structure ensures that they will orientate themselves parallel to the surface that is being worked on. This effect is enhanced with increasing pressure. The flat particles sliding between the surface and the deposits remove the latter with care and along a broad surface, thus reducing the abrasive effect.

Yet this effect is impeded by the creation of spandrels between single fused perlite which do not break down. As they are three-dimensional, the build up of such spandrels can lead to more scratching on the surfaces that they contact, and problematically these do not fall flat under load. Hence this will increase the abrasivity of formulations incorporating such particles.

Surprisingly we have found that perlite can be added to an oral composition comprising chalk, and do not increase the composition's overall abrasivity. This is achieved through a surprising interaction between the perlite and the chalk. In an oral composition the chalk binds to and smoothens any jagged-edges of the perlite spandrels. Consequently the addition of perlite to an oral composition comprising chalk increases the polishing capabilities of that oral composition while not increasing its abrasivity.

Therefore the perlite particles in combination with the chalk particles in the oral composition are far less likely to damage surfaces such as teeth and soft tissue that they contact in the oral cavity. Perlite particles in combination with the chalk particles in the oral composition significantly enhance the cleaning potential of the oral composition so that in combination they provide for improved polishing without causing damage due to excessive abrasion. The term oral composition is known in the art and encompasses a dentrifice, toothpaste, pastille, gum, lozenge, mouthwash and others.

The perlite in the oral composition according to the invention may be any perlite or mixtures of perlite commonly known in the art. It can be crude or non-expanded perlite. Preferably it is expanded perlite which has been quickly heated to above 871°C in order to allow for expansion and then subsequently cooled.

The perlite preferably has an abrasion value of 125 to 150 using RDA, (J Dent Res 37: 1060-1068, 1958 or J Dent Res 55:563 to 573, 1976.). Preferably the perlite is E 50 perlite available from Worldminerals Italia srl., Alzaia Trento 6, 20094 Corsico, Milano, Italy.

The weight median particle diameter of the perlite can be from 10 µm to 50 µm. Preferably the weight median particle diameter is from 20 µm to 45 µm. Most preferably the weight median particle diameter of the perlite is from 30 µm to 40 µm.

Preferably 90% by weight of the perlite has a particle diameter less than 70 µm to 110 µm. Preferably 10% by weight of the perlite has a particle diameter less than 7 µm to 10 µm. The particle size distribution of the perlite is determined using laser light scattering (Malvern 3600E, 300mm Lens).

The oral composition comprises 0.5% to 5% by weight of the oral composition of perlite.

The perlite composition preferably contains SiO₂ 70-75% w/w, Al₂O₃ 10-15% w/w, Fe₂O₃ 0.5-1% w/w, Na₂O 4-5% w/w, K₂O 7-9 % w/w, CaO 0.2-0.4% w/w, MgO 0.05-1.5% w/w, TiO₂ 0.05-1.5% w/w.

Surprisingly the addition of chalk to an oral composition comprising perlite reduces the negative impact perlite can have on an oral composition. Perlite comprises a number of different metals which would ordinarily compromise the stability of some of the important ingredients in an oral composition. Ingredients such as sodium monofluorophosphate which is the fluoride of choice with chalk pastes. Yet surprisingly the stability of the sodium monofluorophosphate is not compromised by the presence of the various metals in the perlite.

The oral composition comprises chalk at from 25% to 45% by weight of the oral composition.

Chalk comprises calcium carbonate, the calcium carbonate in an oral composition leaches carbonate ions. These carbonate ions in turn react with, and as a consequence, mop up metal ions leached from the perlite. Surprisingly this interaction reduces the negative impact the use of relatively high levels of perlite has on an oral composition.

Chalk is also chemically more reactive than alternative abrasives that can be used in oral compositions, such as silica. Therefore these properties of chalk prevent the leached metal ions from exerting a negative effect on an oral composition comprising perlite and chalk.

The oral composition comprises chalk as fine ground natural chalk.

Fine ground natural chalk can be obtained from naturally occurring limestone or marble deposits, which has formed over a period of millions of years before being milled. The milling used may be ball milling followed by sieving followed by a selection of those characteristics which are desired. Fine ground natural chalk may also be modified chemically or physically by coating during milling or after milling, by heat treatment. Typical coatings include magnesium stearate or oleate. The morphology of the fine ground natural chalk may also be modified by the milling process by using different milling techniques, for example ball milling, air-classifier milling and spiral jet milling. By fine ground natural chalk is meant chalk which is obtained by milling limestone or marble deposits and not chalk which has been synthetically precipitated.

Fine ground natural chalk is free from hard and soft clumps of chalk which can interfere with the polishing effect of the perlite. Therefore fine ground natural chalk can smooth the jagged-edges of the perlite in away that is unobtainable with other abrasives such as for example silica. Surprisingly the use of smaller particles results in less interference with the perlite edges that have the beneficial polishing qualities.

Preferably the supplier of the chalk is Omya and the product is preferably 5AV. Specifically the product may be Omya 10 micron. **For compositions of the invention** 90% by weight of the chalk has a particle diameter less than 20 µm. Preferably 50% by weight of the perlite has a particle diameter less than 4 µm to 8 µm.

Alternatively the supplier of chalk is Wolkchem and the product is Addon 1015. 90% by weight of the chalk has a particle diameter less than 20 µm. Preferably 50% by weight of the perlite has a particle diameter less than 4 µm to 8 µm. The BET surface area of the fine ground natural chalk may range from 0.5 to 3 m²/g.

According to another embodiment of the invention there is provided an oral composition as claimed in any preceding claim whereby the pH of the oral composition is 8 to 14.

In a preferred embodiment the pH of the oral composition is 9 to 13, preferably from 10 to 12. Most preferably the pH of the oral composition is 11.

Increasing the pH in an oral composition comprising perlite reduces the leaching of metal ions from the perlite. This means that the negative impact that these ions can have on the oral composition such as reaction with sodium monofluorophosphate, colouration, etc. is reduced.

The oral composition may have a relative dental abrasion (RDA) of 140 to 170.

The relative dental abrasion is 150 to 160, preferably from 152 to 158. Most preferably the relative dental abrasion is 155.

The abrasivity of a toothpaste is measured according to a protocol described in the Journal of Dental Research (1976) 55 (4), 563. This describes how the Relative Dental Abrasion (RDA) is evaluated.

The oral composition may have a percentage polish of 60 to 95.

Preferably the percentage polish is 70 to 90. Most preferably the percentage polish is 75 to 85.

By percentage polish is meant the percentage polishing of each disk. The procedure whereby the percentage polishing of each disk is calculated is discussed in the following paragraphs.

Tests to assess the polishing efficacy are known in the academic literature for example J.G. Masters, A Zaccagnino, R. Heu and M. Stranick. J Dent. Res. 81 Spec Iss A Abs. 2152 (2002). The polishing test used is conducted on hydroxyapatite (HAP) discs. This material was selected as it has similar properties to enamel and is available in a suitable disc format. Prior to brushing with the test toothpaste, the discs were pre-polished to a set gloss value using abrasive papers. The test assesses the polishing properties of the paste by measuring the gloss value of the HAP tile following brushing. Gloss measurements are taken using a Sheen Tri-microgloss meter set at 60deg/60deg (incident/measured angle).

Four HAP discs were used per product tested and each tile was brushed on the WIRA brushing machine (Goodbrand Jeffrys Mark III abrasion tester), using Lissajous figures. A weight of 375g was applied to each disc, brushing speed used was 150rpm, and a standard medium head toothbrush was used. Gloss measurements were taken at 1000, 5000, 10,000 and 15,000 strokes. Four measurements were taken per tile at each time point, using a template so that the measurements where taken at the same point each time.

Toothpaste slurry was made up for each product tested as follows, with 1 part paste, 1 part distilled water (Millipore water) and 1 part SCMC diluent. Whereby the SCMC diluent contained 5% Glycerol, 0.5% SCMC 7m, 0.01% Formalin, and 94.49% Millipore water.

The slurry was mixed using the heidolph end over mixer for 30 minutes ensuring that all paste was thoroughly mixed before use in the study.

The percentage polishing for each disk was calculated by dividing the average gloss value for the disk after brushing by the average baseline gloss value for the same disk and multiplying by 100. This then results in the figures as detailed in Experiment 2 or the percentage polish. The results are detailed in Experiment 2 as polishing percentages per disk. Statistical analysis was done using Excel software, and showed significant differences using Student's t-test to p<0.05.

According to another aspect of the invention there is provided the use of perlite, present in an oral composition, for polishing teeth.

The oral composition according to the invention comprise further ingredients which are common in the art, such as:
antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates;
biomolecules, e.g. bacteriocins, antibodies, enzymes;
flavours, e.g. peppermint and spearmint oils;
proteinaceous materials such as keratin and collagen;
preservatives;
opacifying agents;
colouring agents;
pH-adjusting agents;
sweetening agents;
pharmaceutically acceptable carriers, e.g. starch, sucrose, water or water/alcohol systems;
surfactants, such as anionic, nonionic, cationic and zwitterionic or amphoteric surfactants;
particulate abrasive materials such as silicas, aluminas, dicalciumphosphates, dicalciumphosphate dihydrates, calcium pyrophosphates, hydroxyapatites, trimetaphosphates, insoluble hexametaphosphates and so on, including agglomerated particulate abrasive materials, usually in amounts between 3 and 60% by weight of the oral care composition.
humectants such as glycerol, sorbitol, propyleneglycol, xylitol, lactitol etc, the sorbitol may be present at levels up to 50% by weight of the oral composition;
binders and thickeners such as sodium carboxymethylcellulose, xanthan gum, gum arabic as well as synthetic polymers such as polyacrylates and carboxyvinyl polymers such as Carbopol®;
polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate);
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, antioxidants for example alpha lipoic acid.

Liposomes may also be used to improve delivery or stability of active ingredients.

The invention will now be detailed with reference to the following non- limiting experiments.

### Experiment 1

The following oral composition is an example of the present invention.

| Ingredients | Percentage of Total Weight |
|---|---|
| Chalk | 40 |
| Thickening Silica | 3 |
| SLS | 2.6 |
| Perlite | 0.7 |
| Sodium mono-fluoro phosphate | 0.76 |
| TiO2 | 0.5 |
| Formalin | 0.1 |
| Flavour | 1.1 |
| Saccharine | 0.23 |
| Water | Up to 100 |

### Experiment 2

### Relative Dental Abrasion (RDA)

| Ingredients | RDA |
|---|---|
| Fine Ground Natural Chalk 40% of total weight | 150 |
| Fine ground natural chalk 40% of total weight plus perlite 0.7% of total weight | 155 |

### Polishing

| Ingredients | Polishing Percentage per Disk |
|---|---|
| Fine Ground Natural Chalk 40% of total weight | 49.5 |
| Fine Ground natural chalk 40% of total weight plus perlite 0.7% of total weight | 83.4 |

Fine ground natural chalk in an oral composition has a specific relative dental abrasion. This is not significantly increased when perlite is added to the fine ground natural chalk. Yet the combination of fine ground natural chalk and perlite provides for significantly enhanced polishing. These results demonstrate that the combination of fine ground natural chalk with perlite provides for improved polishing while not increasing the relative dental abrasion.

## Claims

1. An oral composition comprising from 25% to 45% by weight of the oral composition of fine ground natural chalk, **characterised in that** the oral composition comprises from 0.5% to 5% by weight of the oral composition of perlite, and **in that** 90% by weight of the chalk has a particle diameter less than 20 µm.

2. An oral composition as claimed in any preceding claim, whereby the pH of the oral composition is 8 to 14.

3. Use of perlite for polishing teeth **characterized in that** the perlite is included in an oral composition as claimed in any preceding claim.

## Patentansprüche

1. Orale Zusammensetzung, die 25 bis 46 %, und zwar auf das Gewicht der oralen Zusammensetzung bezogen, fein gemahlenen natürlichen Kalk aufweist, **dadurch gekennzeichnet, dass** die orale Zusammensetzung 0,5 bis 5 %, und zwar auf das Gewicht der oralen Zusammensetzung bezogen, Perlit aufweist, und dass 90 Gew.-% des Kalks einen Teilchendurchmesser von weniger als 20 µm aufweisen.

2. Orale Zusammensetzung nach einem der vorstehenden Ansprüche, wobei der pH-Wert der oralen Zusammensetzung 8 bis 14 beträgt.

3. Verwendung von Perlit zum Polieren der Zähne, **dadurch gekennzeichnet, dass** in der oralen Zusammensetzung nach einem der vorstehenden Ansprüche Perlit enthalten ist.

## Revendications

1. Composition orale comprenant de 25 % à 45 % en masse de la composition orale de fine craie naturelle broyée, **caractérisée en ce que** la composition orale comprend de 0,5 % à 5 % en masse de la composition orale de perlite, et **en ce que** 90 % en masse de la craie présentent un diamètre de particule inférieur à 20 µm.

2. Composition orale selon l'une quelconque des revendications précédentes, dans laquelle le pH de la composition orale est de 8 à 14.

3. Utilisation de perlite pour le polissage des dents, **caractérisée en ce que** la perlite est incluse dans une composition orale selon l'une quelconque des revendications précédentes.
